Europäisches Patentamt

European Patent Office

Office européen des brevets

(11) Publication number: **0 263 190**
**A1**

## (12) EUROPEAN PATENT APPLICATION

(21) Application number: **86113609.1**

(22) Date of filing: **02.10.86**

(51) Int. Cl.4 **A61B 5/02 , G01L 9/00**

(43) Date of publication of application:
**13.04.88 Bulletin 88/15**

(84) Designated Contracting States:
**AT BE CH DE ES FR GB GR IT LI LU NL SE**

(71) Applicant: **PPG HELLIGE B.V.**
**I.B.C.-Weg 1**
**NL-5683 PK Best(NL)**

(72) Inventor: **Kuypers, Martinus Henricus**
**Schoolstraat 53**
**NL-5561 AH Riethoven(NL)**

(74) Representative: **Patentanwälte TER MEER -**
**MÜLLER - STEINMEISTER**
**Mauerkircherstrasse 45**
**D-8000 München 80(DE)**

(54) **Pressure transducer for medical applications.**

(57) A disposable catheter pressure transducer consists of a plastic support member (4, 22) for a semiconductor pressure transducer (8), with said support member being held by a metal tube (1) which with a portion (2) of reduced diameter is inserted and fixed to the distal end of the catheter tube (3). An internal tube (12) feeding a reference pressure to the bottom side of a semiconductor pressure measuring membrane (9) simultaneously surrounds the electrical connecting wires (13) for the transducer chip and a reinforcement wire (14) which gives the catheter the necessary stability for placing the catheter tip at the desired location within the patient.

Fig.1

EP 0 263 190 A1

## Pressure Transducer for Medical Applications

The invention is directed to a pressure transducer which can easily be manufactured at low cost so that it might be disposed of after use instead of being cleaned and sterilized for further use. The new transducer therefor should be suited for mass production with well-known and well-established manufacturing methods. On the other hand the pressure transducer as a medical measuring device needs to be reliable and accurate. The new transducer preferably will be mounted at the tip of a catheter.

In EP-A 0 115 548 a catheter pressure transducer is described where the sensor consists of a support having the shape of a cylinder cut-through in longitudinal direction with a semiconductor sensor element being provided on the cut surface. The diameter of the cut cylinder is smaller than the internal diameter of the catheter. The support is eccentrically provided within the catheter such that it engages that side of the internal wall of the catheter which includes an opening to the outside and is sealed against the catheter. A space sickle-shaped in cross-section is formed between the sensor and that internal wall of the catheter which is opposite the opening, and that space extends along the entire length of the sensor. This known catheter sensor can be attached to and used with catheters of different diameter. The sickle-shaped space may be used for the supply of medicine or the extraction of body liquid. The catheter wall in the region of the sensor is formed by a metal tube comprising the lateral opening and being fixed to the distal end of a flexible catheter tube.

US-A 42 74 423 shows a catheter tip pressure transducer with a pressure-sensitive diaphragm with strain gauges formed thereon. The pressure sensor is mounted on an insulating supporting member, and a protecting member made of silicon rubber fills the hollow distal end portion of the flexible catheter tube. A further catheter transducer of this type is shown in US-A 41 91 193 where the flexible catheter tube is reinforced by a stainless steel insert, and the catheter itself is only used as a guide and tube for the electrical connecting wires of the sensor tip. Another pressure measuring catheter having a piezoelectric pressure transducer is known from US-A 44 56 013 where again the transducer is inserted in a portion of the catheter tube itself which in the area of the transducer is reinforced by means of an inserted tube.

The present invention by using a metal tube in the area of the transducer provides more space for the transducer than it could be obtained when putting the transducer inside the plastic catheter tube. By this provision therefor the diameter of the tube can be reduced without reducing the space for the semiconductor transducer. The provision of a reinforcement wire makes it easier to move the catheter tip to the correct position without impairing the flexibility of the catheter tube wall itself. Since the reinforcement wire is provided within the internal tube, it is isolated from the residual internal space of the catheter which may be used for supplying medicine to the patient or for drawing body liquid from the patient. With the supporting member for the semiconductor transducer forming itself the cover or closure of the distal end of the metal tube, a very compact structure is achieved which requires few steps for assembly and is well-suited for miniaturization.

Further details and improvements of the invention are described in the subclaims. Some embodiments will be ex plained with reference to the enclosed drawings in which

Fig. 1 shows a longitudinal section through a first embodiment of the transducer catheter;

Fig. 2 shows a cross-section through the sensor tip along line II-II;

Fig. 3 shows a cross-section along line III-III;

Fig. 4a and 4b show examples of the cross-section of a multi-functional cable used for connecting electrically and hydraulically the transducer to the proximal end of the catheter;

Fig. 5 shows a second embodiment of the catheter tip transducer;

Fig. 6 shows as a third embodiment a modified version of the Figure 5 type of transducer catheter.

Fig. 7 and 7a show a fourth embodiment.

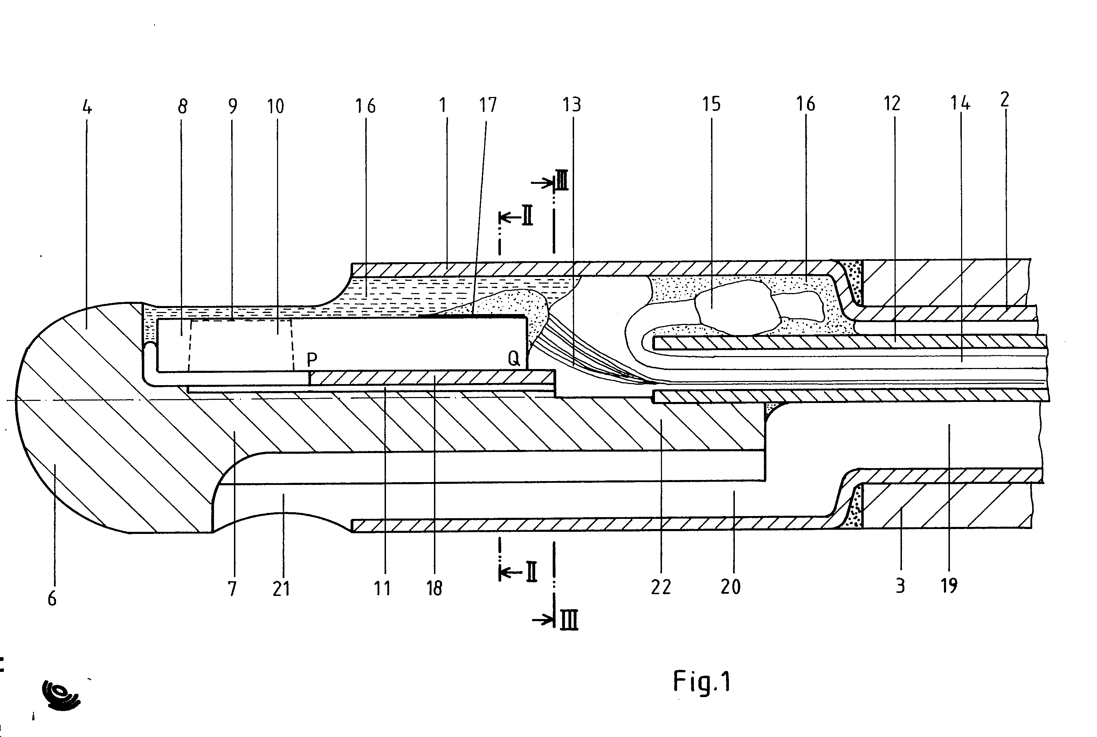

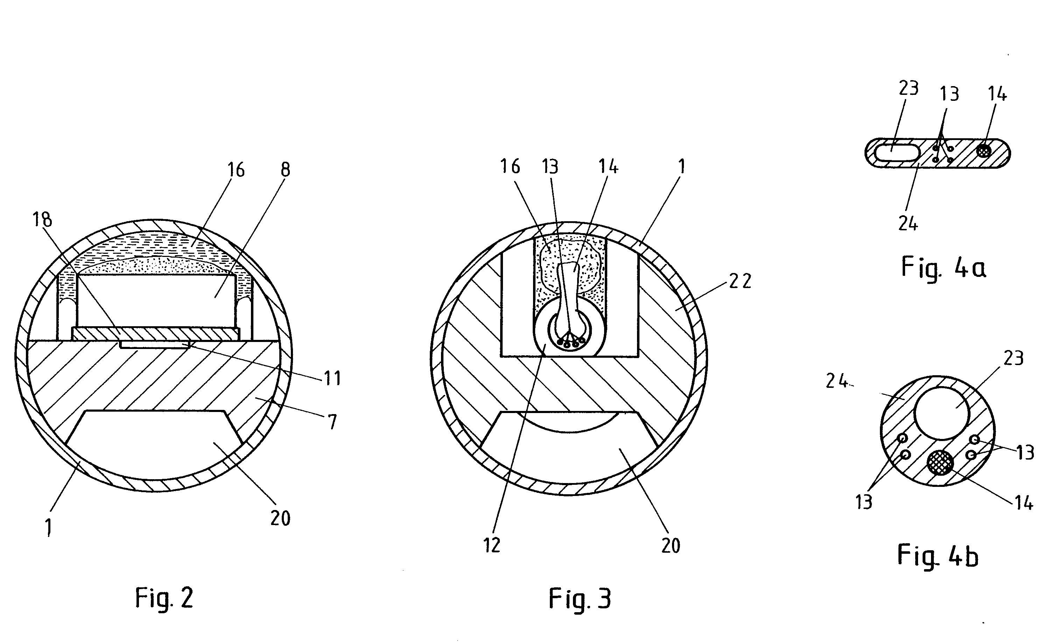

In Figure 1 a metal tube 1 preferably of stainless steel has a proximal end portion 2 of reduced diameter and with this portion is inserted into the distal end of the biomedical catheter tube 3. A preformed support member 4 consisting of plastic material with a high module of elasticity covers the distal end 5 of metal tube 1. This support member has a semispherical end portion 6 forming the tip of the catheter and an extension 7 of semicircular cross-section (see Figure 2) which forms the support portion for the semiconductor transducer 8. Support member 4 is preferably made of composite material such as thermoplastics filled with glass or carbon fiber or a thermosetting plastic like epoxy-novolac. The material of support member 4 is chosen such that epoxy adhesive reliably adheres to the support member. Instead of using plastic material ceramic material or glass preforms formed in the green state by dry pressing and isostatic pressing and subsequent firing may be used. As Figure 1 shows, the transducer chip is supported by a support member 18 along a surface extending from P to Q which lies outside the actual pressure sensing portion 9 formed by a semiconductor diaphragm with strain gauges formed therein. A recess 10 below this sensing diaphragm 9 permits a reference pressure from channel 11 to be supplied to the bottom side of diaphragm 9. This channel 11 is connected to the inside of internal tube 12 which preferably consists of polytetrafluorethylene or polyethylene and at its end portions is made adhesible by etching or other well-known processes. This internal tube 12 simultaneously surrounds the electrical connecting wires 13 which connect the transducer 8 to a monitoring circuit and furthermore covers a reinforcement wire 14. This wire extends along the total length of the catheter and with its distal end 15 is fixed by adhesive 16 or otherwise to the support member 4 and the metal tube 1. This axial reinforcement wire consists of a material with a low module of elasticity and may consist for instance of polyaramid or a multifilament wire coated with polyurethane. The reason for supporting transducer element 8 only at surface portions outside the actual sensing portion 9 is to prevent mechanical stress, as e.g. generated by thermal expansion at the silicon adhesive support interface from moving from the support member to the sensing portion. This portion 9 at the left part of chip 8 projects touch-free like a cantilever over supporting member 4 so that any mechanical stress generated by bonding the chip with expoxide to the plastic supporting member 4 cannot reach the sensing portion. The ends of wires 13 are connected to the semiconductor chip 8 by means of contact pads 17 and are fixed to them preferably by ultrasonic bonding.

The internal space 19 of catheter tube 2 is in communication with the outside via a channel 20 formed between an axial recess of support member 4 and metal tube 1 and by a hole 21 left between support member 4 and metal tube 1. Support member 4 and transducer 8 are hermetically sealed within metal tube 1 by means of silicon rubber and epoxide 16.

For manufacturing and assembling the transducer catheter the transducer chip 8 is placed on supporting member 4 and fastened to it by means of an adhesive or entectic bonding. Internal tube 12 with the electrical wires 13 and the reinforcement wire 14 are prepared and fastened to portion 22 of supporting member 4. As a next step the end portions of wires 13 are bonded to the contact pads 16 on semiconductor substrate 8. Finally support member 4, metal tube 1 and catheter tube 3 are mounted together and sealed.

Whereas in Fig. 1 the electrical wires 13 and the reinforcement wire 14 are loosely lying in internal tube 12, Figs. 4a and 4b show two possible improvements of the internal tube with the inserted reinforcement wire and electrical wires. In Fig. 4a this multifunctional cable has a flat cross-section like a so-called band cable, whereas in Figure 4b it is a cable of circular cross-section. In both cases a channel 23 forms the conduit for the reference pressure which is fed to the bottom side of membrane 9. Electrical wires 13 are embedded into the cable, and the reinforcement wire 14 is also included in the same multifunctional cable 24, which as the internal tube 12 may be made of PTFE or PE.

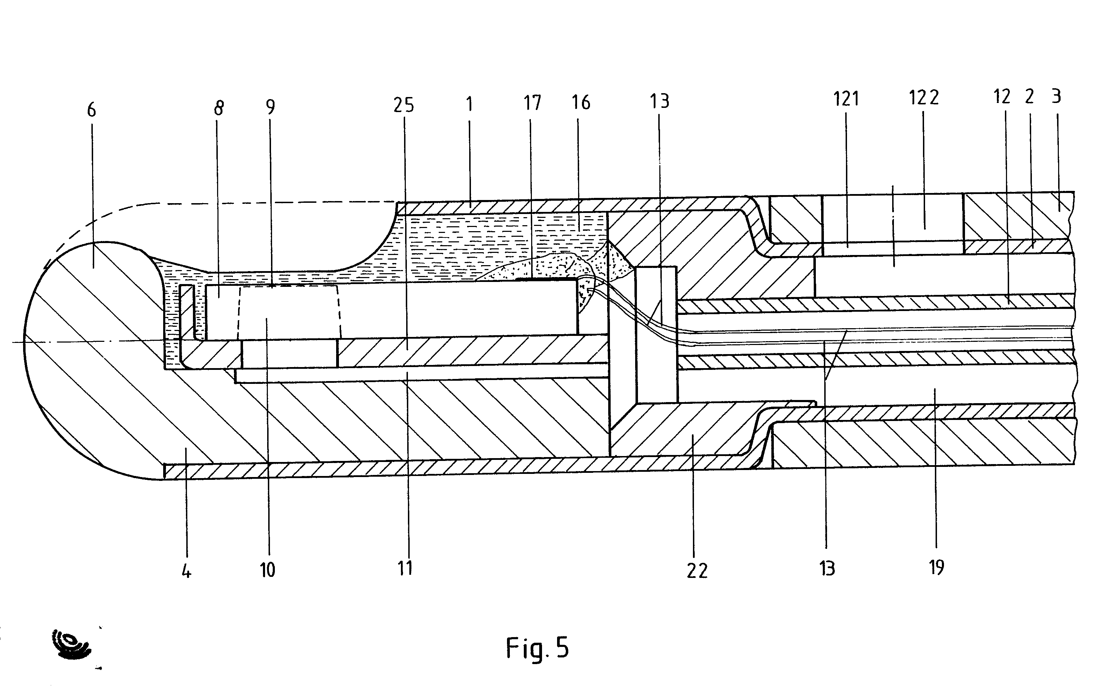

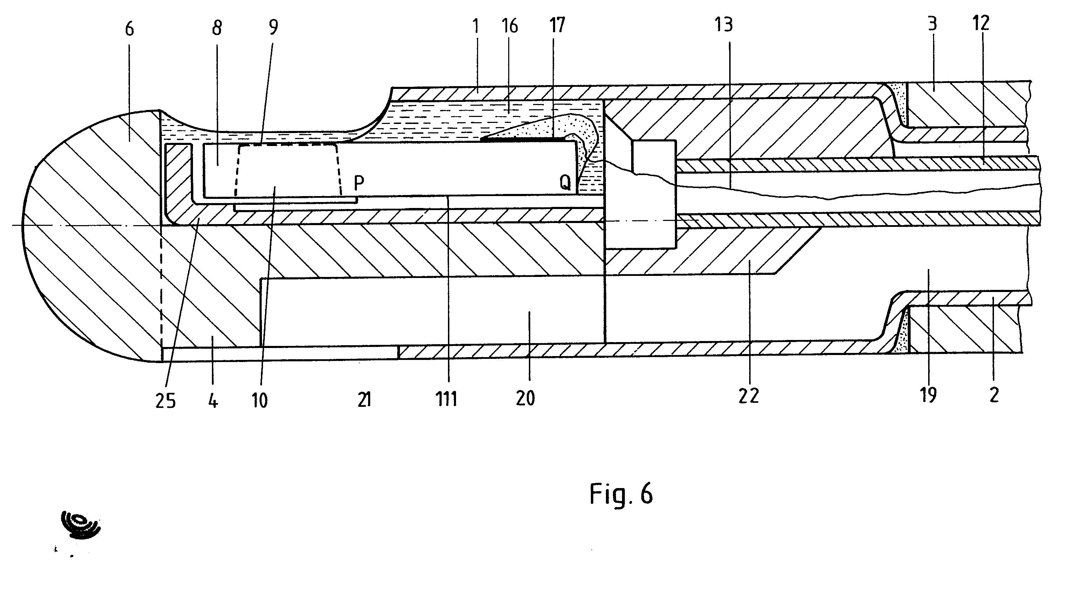

Whereas in Figure 1 the semiconductor transducer 8 is directly supported by supporting member 4, the embodiments shown in Figures 5 and 6 show a different type of support. In this case a separate metal frame 25 is provided on supporting member 4 and is made of a material having almost the same coefficient of linear thermal expansion as the semiconductor material or transducer 8. In this way again any mechanical stress generated by thermal deformation of supporting member 4 is prevented from influencing semiconductor transducer 8 and in particular its sensor region 9.

In Figure 5 the two-piece support member 4, 22 completely fills the metal tube 1, and the opening 121 to the outside is provided in end portion 2 of metal tube 1 and is in alignment with a corresponding opening 122 in the distal end of catheter tube 3. Channel 11 provides communication between the bottom surface of diaphragm 9 and the internal side of internal tube 12. Reinforcement wire 14 (not shown) is fastend with its free end to portion 22 of the supporting member.

In Figure 6 opening 21 to the outside is again provided in the distal end portion of metal tube 1, and channel 111 is formed by a recess within metal frame 25. Wires 13 are shown schematically and of course are isolated from each other. Also in the Figure 5 and Figure 6 embodiment a multifunctional cable 24 may be used instead of internal tube 12, reinforcement wire 14 and electrical wires 13.

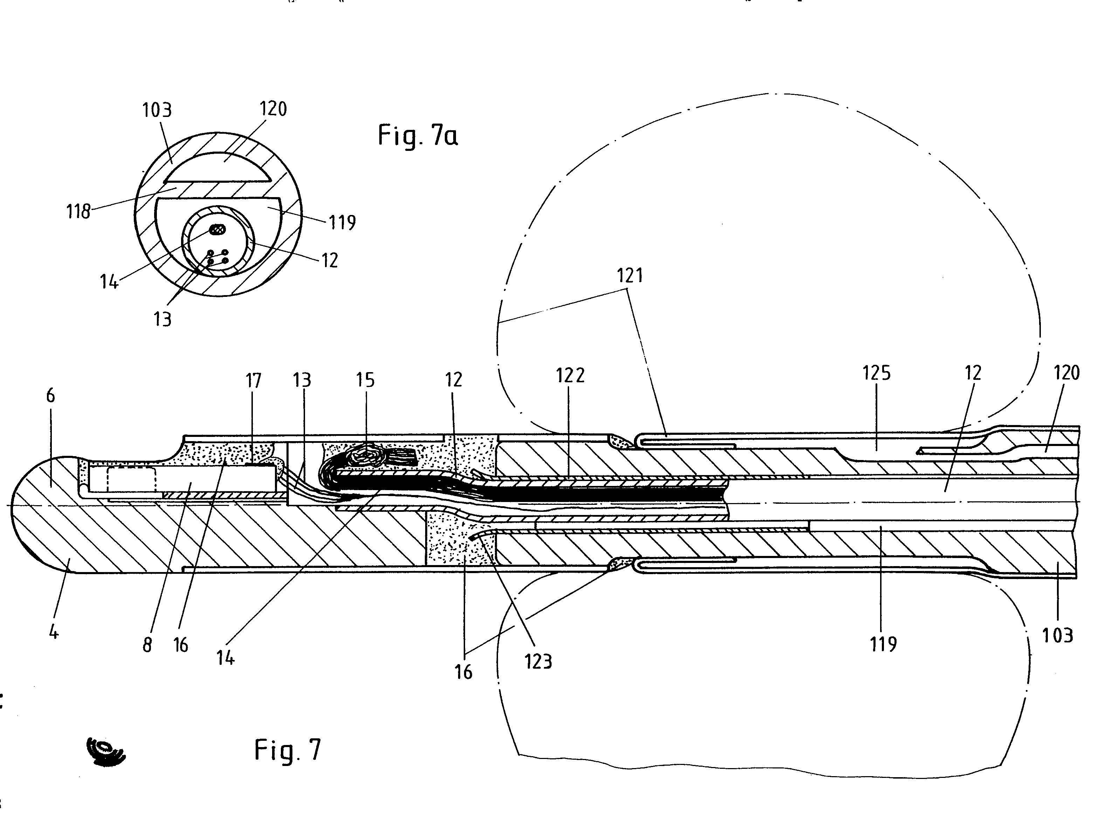

Whereas in the preceding embodiments the catheter tube 3 had a concentric lumen 19, Fig. 7 shows part of another embodiment where catheter tube 103 has a non-symmetric lumen 119 forming the main channel and has a second channel 120 with both channels being separated from each other by a wall 118. Fig. 7 shows how a pressure transducer according to the invention can be fastened to such a catheter having a non-symmetric lumen. Catheters of this type are e.g. balloon cathethers having an extendable balloon 121. Other catheters with non-symmetric lumen are so-called Swan-Ganz catheters and thermodilution cathethers.

In this case a metal guiding tube 122 is inserted into the free lumen 119 of the cathether plastic tube 103. Guiding tube 122 has a funnel-shaped distal end 123, and the internal tube 12 or a corresponding multifunctional cable 23, 24 extends through the guiding tube. The metal tube 1 is fixed to and sealed against the catheter plastic tube 3, the internal tube 12 and against the guiding tube 122 by means of an epoxide or silicon rubber fill 124. Second channel 120 serves for supplying an expansion fluid along the cathether into balloon 121 via opening 125.

In Fig. 7a a cross-section of a modification of such a non-symmetric catheter 103 is shown, whereat lumen 119 is provided non-coaxially within catheter 103. Also in this case a metal guiding tube 122 is useful.

## Claims

1. Pressure transducer comprising

a) a metal tube (1) fixed to the distal end of a catheter plastic tube (3);

b) a support member (4, 22) for a semiconductor pressure transducer (8) provided in said metal tube and partially exposed to the pressure under measurement;

c) an internal tube (12, 23, 24) of smaller diameter than the catheter tube, said internal tube forming a supply channel for a reference pressure fed to the bottom side of the membrane (9) of the semiconductor pressure transducer (8);

d) isolated electrical connecting wires (13) guided through said internal tube for connecting said transducer to a pressure monitoring circuit;

e) an opening (21, 125) connecting the internal space of the catheter tube (3) outside the internal tube to the outside of the catheter, **characterized in that**

f) the distal end (5) of the metal tube (1) is closed by a rounded portion (6) of the support member (4) made of plastic or ceramics;

g) the wall of the internal tube (12, 24) surrounds an axial reinforcement wire (14), the distal end of which is fixed to the support member (4, 22) as is the distal end of the internal tube, said wire extending along the entire length of the catheter.

2. Transducer according to claim 1, **characterized in that** the metal tube (1) has a proximal end (2) with reduced diameter which is inserted into and fixed to the distal end of the catheter tube (3).

3. Transducer according to claim 2, **characterized in that** the opening (21, 122) extends through said metal tube end portion (2) of reduced diameter and through the end portion of the cathether tube (3).

4. Transducer according to claim 1 or 2, **characterized in that** the opening (21) is formed between the distal end portion of the metal tube (1) and an axial recess of the support member (4, 22), said recess providing a channel (20) which communicates with the internal space of the catheter tube (3).

5. Transducer according to one of the claims 1 to 4, **characterized in that** the support member (4, 22) comprises a semicylindrical portion (7) extending from the rounded semispherical end portion (6), with said semicylindrical portion forming at its flat side the support for the semiconductor pressure sensor (8) and being fixed with its proximate end to the internal tube (12, 24) and the reinforcement wire (14).

6. Transducer according to one of the claims 1 to 5, **characterized in that** the supported surface (P - Q) of the semiconductor transducer (8) is apart from the pressure sensing portion (9) of the transducer.

7. Transducer according to claim 6, **characterized in that** the sensing portion (9) of the transducer is provided in a cantilever which is supported on and extends from the support surface (P - Q) of the support member (4, 22).

8. Transducer according to one of the claims 1 to 7, **characterized in that** a metal frame (25) is inserted between the support member (4, 22) and the supported surface of the semiconductor transducer (8), with said metal frame having about the same coefficient of thermal expansion as the semiconductor material.

9. Transducer according to claim 1 or one of the claims 5 to 8 when dependent on claim 1, **character ized in that**

a) a metal guiding tube (122) is inserted into the free lumen (119) of the catheter plastic tube (103);

b) the guiding tube (122) has a funnel-shaped distal end (123);

c) the internal tube (12, 24) extends through the guiding tube;

d) the metal tube (1) is fixed to and sealed against the catheter plastic tube (3), the internal tube (12, 24) and the guiding tube (122, 123) by means of an epoxide or silicon rubber fill (124).

10. Transducer according to one of the claims 1 to 9, **characterized in that** the internal tube (23) together with the electrical connecting wires (13) and the reinforcement wire (14) forms a prefabricated multifunctional cable (24) of flat or circular cross-section.

Fig.1

Honeywell

0 263 190

8C 113 609.1

781006 00

Fig. 4a

Fig. 4b

Fig. 2

Fig. 3

Fig. 5

0 263 190

0 263 190

Fig. 6

Fig.7a

Fig.7

Honeywell

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y,D | EP-A-0 115 548  (HONEYWELL AND PHILIPS MEDICAL ELECTRONICS B.V.)<br>* Abstract; page 2, line 21 - page 3, line 11; page 3, lines 24-35; figures 1,2 * | 1,5 | A 61 B    5/02<br>G 01 L    9/00 |
| A | | 3 | |
| Y,D | EP-A-0 074 114  (BROWN UNIVERSITY RESEARCH FOUNDATION)<br>* Abstract; page 5, line 17 - page 6, line 11; page 6, lines 19-20; page 7, lines 5-14; figures 1-3,8 * | 1,5 | |
| A | | 2,8,9 | TECHNICAL FIELDS SEARCHED (Int. Cl 4) |
| A,D | US-A-4 274 423  (MIZUND et al.)<br><br>* Abstract; column 4, lines 7-27; column 5, lines 9-19,44-48; column 5, line 66 - column 6, line 4; column 6, lines 58-65; figures 3-7 * | 1,5,6,8 | A 61 B<br>G 01 L<br>A 61 M |
| A | US-A-3 088 323  (WELKOWITZ AND TRAITE)<br>* Column 1, lines 9-12,61-71; figure 1 * | 1,6,7 | |

The present search report has been drawn up for all claims

| Place of search | Date of completion of the search | Examiner |
|---|---|---|
| THE HAGUE | 01-06-1987 | FERRIGNO, A. |